# EUROPEAN PATENT APPLICATION

(11) **EP 3 583 897 A1**
(43) Date of publication of application: **25.12.2019**
(21) Application number: 17896727.9
(22) Date of filing: 25.12.2017
(51) Int. Cl.: A61B 5/08, G01N 33/497

(54) **BREATH MEASUREMENT DEVICE**

(30) Priority: 15.02.2017 JP 2017025845
(71) Applicant: PHC Holdings Corporation, Tokyo 105-8433 (JP)
(72) Inventor: KAWAMOTO, Toru, 2131-1, Minamigata, Toon-shi, Ehime 791-0395 (JP); SAWADA, Tsuyoshi, 2131-1, Minamigata, Toon-shi, Ehime 791-0395 (JP); TAMAI, Norihiko, 2131-1, Minamigata, Toon-shi, Ehime 791-0395 (JP)
(74) Representative: Grünecker Patent- und Rechtsanwälte PartG mbB
(86) International application number: PCT/JP2017/046372
(87) International publication number: WO 2018/150738

(57) **Abstract**

A flow rate detector **(12)** of an exhalation measurement device **(1)** includes: a pipe body **(20)** having a flow inlet **(21)** at a first end into which exhalation flows, and a flow outlet **(22)** at a second end out of which exhalation flows; and a differential pressure sensor **(23)** connected to the pipe body **(20).** The pipe body **(20)** includes: a partition gasket **(28)** which partitions the inside of the pipe body **(20)** into a flow-in space **(32)** of exhalation and a flow-out space **(33)** of exhalation; a connection aperture **(35)** being provided at the flow-in space **(32)** and connecting the differential pressure sensor **(23)** to the flow-in space **(32);** a connection aperture **(36)** being provided at the flow-out space **(33)** and connecting the differential pressure sensor **(23)** to the flow-out space **(33);** and an air duct **(29)** of an elongated shape, extending through the partition gasket **(28)** and allowing the flow-in space **(32)** and the flow-out space **(33)** to communicate with each other.

## Description

### TECHNICAL FIELD

The present invention relates to, for example, an exhalation measurement device to be used when e.g. detecting asthma, or checking pulmonary function.

### BACKGROUND ART

A conventional exhalation measurement device would include: a measurement apparatus main body into which exhalation is blown; a chamber to temporarily retain the exhalation that has been blown into the measurement apparatus main body; a pump to allow the exhalation within the chamber to be supplied to a measurement section; a control section to control operation of the pump; and a flow rate detector to detect a flow rate of the exhalation that is supplied by the pump.

In other words, when measuring the ammonia or the like that is contained within exhalation, after the exhalation is once retained in the chamber, this exhalation in the chamber is sucked out by the pump, so as to be supplied to the measurement section.

In order to take a measurement of this exhalation, the exhalation needs to be supplied to the measurement section with a predetermined flow rate (i.e., an amount of exhalation to flow per unit time). Thus, as the flow rate detector detects the flow rate of the exhalation that is supplied by the pump, the pump may be controlled on the basis of this detected value, whereby the exhalation is supplied to the measurement section with a predetermined flow rate (see, for example, Patent Document 1).

### SUMMARY OF INVENTION

### TECHNICAL PROBLEM

[Patent Document 1] Japanese National Phase PCT Laid-Open Publication No. 2010-509586

### SOLUTION TO PROBLEM

A problem in the above conventional example is that the measurement accuracy for exhalation may become lower.

Specifically, the flow rate detector to detect a flow rate of the exhalation that is supplied by the pump measures a pressure of the exhalation at the upstream side and at the downstream side of the exhalation, and detects a flow rate of the exhalation by using a pressure difference therebetween.

However, usually, such a pressure difference in the exhalation is so small that it is difficult to measure the pressure difference in the exhalation with a sufficient accuracy, and this has led to a low detection accuracy of the flow rate of the exhalation that is detected by using the pressure difference.

Thus, when the detection accuracy for the flow rate of the exhalation is low, the pump cannot be appropriately controlled, so that exhalation cannot be supplied to the measurement section with a predetermined flow rate, thereby resulting in a lower measurement accuracy for exhalation.

Accordingly, an objective of the present invention is to enhance the measurement accuracy for exhalation.

In order to attain this objective, an exhalation measurement device according to the present invention includes: a measurement apparatus main body into which exhalation is to be blown; a measurement section to take a measurement of the exhalation; a chamber to temporarily retain the exhalation that has been blown into the measurement apparatus main body; a pump to allow the exhalation in the chamber to be supplied to the measurement section; a control section to control operation of the pump; and a flow rate detector to detect a flow rate of the exhalation that is supplied by the pump to the measurement section.

The flow rate detector includes: a pipe body having a flow inlet at a first end, into which the exhalation flows, and a flow outlet at a second end opposite to the first end, out of which the exhalation flows; and a differential pressure sensor connected to the pipe body.

The pipe body includes: a partitioning member which partitions an inside of the pipe body into a first space at a flow-in side of the exhalation and a second space at a flow-out side of the exhalation; a first connection aperture being provided at the first space side and connecting the differential pressure sensor to the first space; and a second connection aperture being provided at the second space side and connecting the differential pressure sensor to the second space; and an air duct of an elongated shape extending through the partitioning member and allowing the first space and the second space to communicate with each other.

### (EFFECTS OF INVENTION)

With an exhalation measurement device according to the present invention, a pipe body of a flow rate detector includes a partitioning member which partitions the inside into a first space at a flow-in side of the exhalation and a second space at a flow-out side of the exhalation, and an air duct of an elongated shape extending through the partitioning member and allowing the first space and the second space to communicate with each other. Therefore, in the inside of the pipe body, the exhalation passes through the thin and long air duct.

This induces an increased pressure difference in the exhalation between the first space and the second space, which will be measured with a sufficient accuracy. Therefore, the detection accuracy for the flow rate of the exhalation as detected by using the pressure difference can be enhanced.

As a result of this, the pump to adjust the flow rate of the exhalation is appropriately controlled, and the exhalation is supplied to the measurement section with a predetermined flow rate, whereby the measurement accuracy for exhalation can be enhanced.

### BRIEF DESCRIPTION OF DRAWINGS

[FIG. **1**] A perspective view showing the construction of an exhalation measurement device according to Embodiment 1 of the present invention.
[FIG. **2**] FIG. **1** is a control block diagram of the exhalation measurement device.
[FIG. **3**] FIG. **1** is an exploded perspective view of a flow rate detector of the exhalation measurement device.
[FIG. **4**] An upper plan view of the flow rate detector of the exhalation measurement device in FIG. **1****.**
[FIG. **5**] A side view of the flow rate detector of the exhalation measurement device in FIG. **1****.**
[FIG. **6**] A cross-sectional view, taken at line **A-A** and viewed in the direction of arrows in FIG. **4****.**
[FIG. **7**] A cross-sectional view, taken at line **B-B** and viewed in the direction of arrows in FIG. **5****.**
[FIG. **8**] A cross-sectional view showing the construction of a main portion of the exhalation measurement device in FIG. **1****.**

### DESCRIPTION OF EMBODIMENTS

Hereinafter, with reference to the drawings as necessary, embodiments will be described in detail. Note however that unnecessarily detailed descriptions may be omitted. For example, detailed descriptions of what is well known in the art or redundant descriptions of what is substantially the same constitution may be omitted.

This is to avoid lengthy description, and facilitate the understanding of those skilled in the art.

The accompanying drawings and the following description, which are provided by the present inventors so that those skilled in the art can sufficiently understand the present disclosure, are not intended to limit the scope of claims.

Hereinafter, an embodiment of the present invention will be described with reference to the drawings.

### (Embodiment 1)

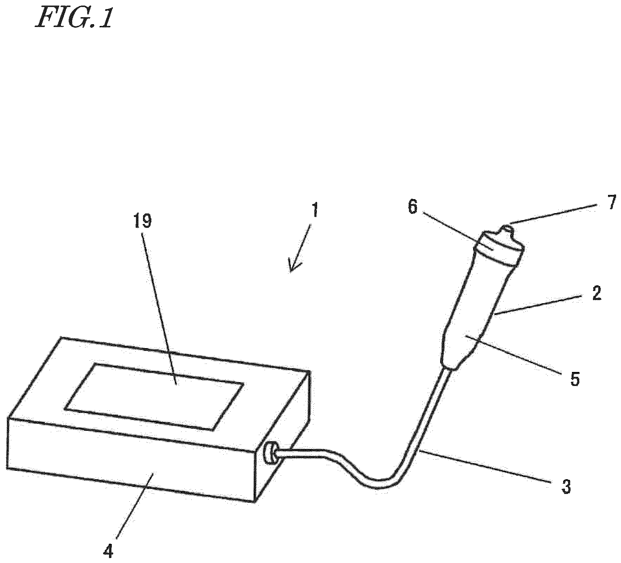

FIG. **1** shows an exhalation measurement device **1** to be used when e.g. detecting asthma, or checking pulmonary function.

The exhalation measurement device **1** according to the present embodiment measures an amount of nitrogen monoxide that is contained in exhalation (i.e., a nitrogen monoxide concentration within exhalation), for example. In the exhalation measurement device **1,** a handle section **2** with which to perform sucking and blowing of exhalation is connected to the measurement apparatus main body **4** via a tube **3.**

The handle section **2** includes a handle section main body **5** and a mouthpiece **6** that is mounted above the handle section main body **5.**

As a user sucks in for exhalation, with his or her mouth being placed on an exhalation opening **7** of the mouthpiece **6,** atmospheric air is taken into the handle section main body **5** through an intake port (not shown). From the atmospheric air that has been taken in, nitrogen monoxide is removed by a nitrogen monoxide removing agent that is contained in a filter section (not shown).

As the user blows exhalation into the exhalation opening **7,** the exhalation that has been blown in passes through the tube **3** and flows into the measurement apparatus main body **4** in FIG. **1****.** The exhalation contains nitrogen monoxide that has occurred in the airway of the user.

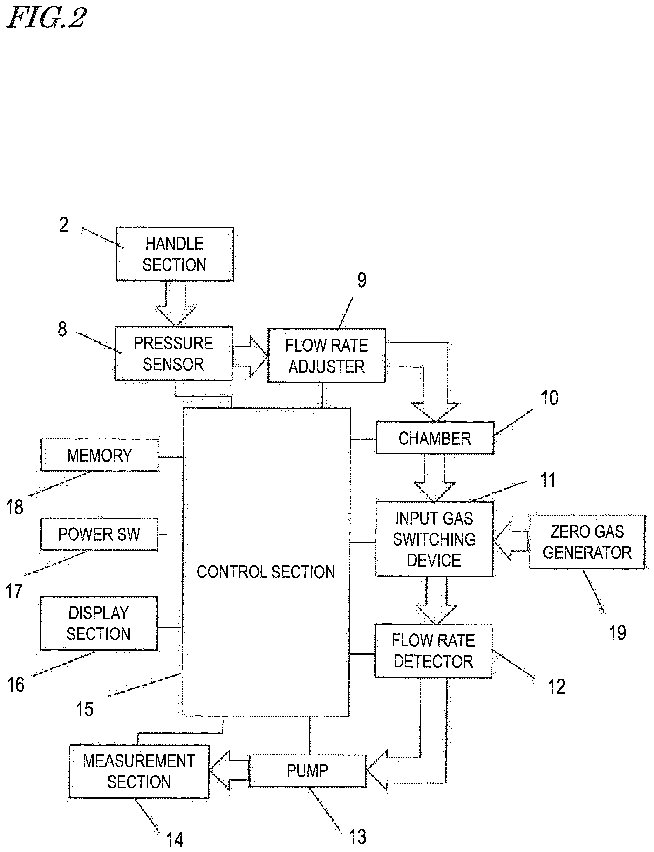

FIG. **2** is a control block diagram of the measurement apparatus main body **4.**

As shown in FIG. **2****,** the following are provided in the measurement apparatus main body **4:** a pressure sensor **8** to measure the pressure of the exhalation that is blown in; a flow rate adjuster **9** to adjust the exhalation to a predetermined flow rate (i.e., an amount of exhalation to flow per unit time; e.g., 50ml/second) by using a value from the pressure sensor **8;** and a chamber **10** that temporarily retains the exhalation that has undergone the flow rate adjustment by the flow rate adjuster **9.**

Via an input gas switching device **11** and a flow rate detector **12,** the exhalation within the chamber **10** is sent from a pump **13** to a measurement section **14.** The measurement section **14** takes a measurement of the exhalation. Note that the pressure sensor **8,** the flow rate adjuster **9,** the input gas switching device **11,** the flow rate detector **12,** the pump **13,** and the measurement section **14** are electrically connected to a control section **15** as shown in FIG. **2****.** Also electrically connected to the control section **15** are a display section **16,** a power switch **17,** and a memory **18.** The control section **15** controls the respective sections connected thereto, and also controls operation of the pump **13.**

When measuring the nitrogen monoxide contained in the exhalation, after the exhalation is once retained in the chamber **10,** the exhalation in the chamber **10** is sucked out by the pump **13** so as to be supplied to the measurement section **14.** In other words, when the pump **13** operates, the pressure of the exhalation will be smaller at the pump **13** side than at the chamber **10** side; therefore, as shown in FIG. **2****,** the exhalation will flow from the chamber **10** toward the measurement section **14.**

Now, in order to take a measurement of the exhalation, the exhalation needs to be supplied to the measurement section **14** with a predetermined flow rate.

Therefore, the flow rate detector **12** detects a flow rate of the exhalation that is supplied by the pump **13,** and the pump **13** is controlled on the basis of this detected value, whereby the exhalation is supplied to the measurement section **14** with the predetermined flow rate.

This predetermined flow rate is stored in the memory **18** as a predetermined flow rate value that is predefined. Various control programs to be used by the control section **15** are also stored in the memory **18.**

Moreover, a zero gas generator **19** is connected to the input gas switching device **11.** In an exhalation measurement, the input gas switching device **11** switches from connecting between the chamber **10** and the flow rate detector **12** to connecting between the zero gas generator **19** and the flow rate detector **12.**

Hereinafter, a fundamental exhalation measurement operation will be described.

Description herein will begin from a state where the exhalation that has been blown in by the user is already retained in the chamber **10.**

In an exhalation measurement, first, the exhalation in the chamber **10** is measured to obtain an actual measurement value.

Specifically, while the control section **15** controls the pump **13** so that the exhalation will have the predetermined flow rate (e.g., 2 ml/second), the exhalation in the chamber **10** is supplied to the measurement section **14,** via the input gas switching device **11** and the flow rate detector **12.**

At this time, the control section **15** controls the pump **13** on the basis of a detected value of the flow rate of the exhalation as detected by the flow rate detector **12,** whereby the exhalation is supplied to the measurement section **14** with the predetermined flow rate.

The measurement section **14** measures the concentration of nitrogen monoxide that is contained in the exhalation. Through this measurement, an actual measurement value of the exhalation is obtained.

Next, the atmospheric air in the measurement environment is measured to obtain a reference value.

Specifically, while the control section **15** controls the pump **13** and the input gas switching device **11,** the atmospheric air in the measurement environment is taken in via the zero gas generator **19** and the input gas switching device **11,** so as to be supplied to the measurement section **14.**

At this time, the atmospheric air in the measurement environment passes through the zero gas generator **19,** whereby nitrogen monoxide is removed therefrom. The measurement section **14** measures the atmospheric air in the measurement environment from which nitrogen monoxide has been removed. Through this measurement, a reference value is obtained.

Thereafter, the control section **15** compares actual measurement value of the exhalation against the reference value, thereby calculating an amount of nitrogen monoxide that is contained in the exhalation (i.e., a nitrogen monoxide concentration within the exhalation), and causes this value to be displayed on the display section **16.**

In the present embodiment, the detection accuracy for the flow rate of the exhalation as detected by the flow rate detector **12** in FIG. **2** is enhanced, whereby the pump **13** is appropriately controlled on the basis of the detected flow rate of the exhalation, so that the exhalation is supplied to the measurement section **14** with the predetermined flow rate. As a result of this, the measurement accuracy of the concentration of nitrogen monoxide that is contained in the exhalation can be enhanced.

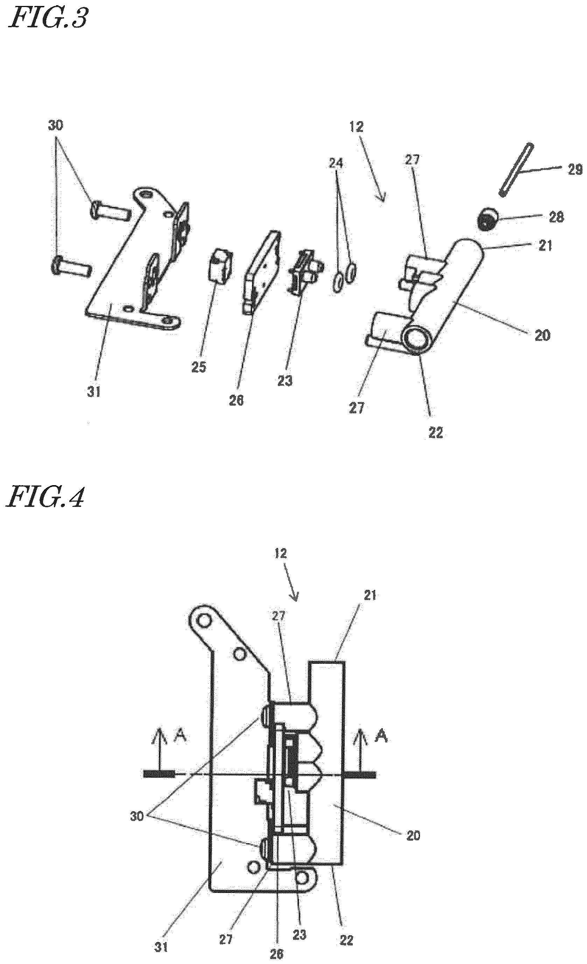

Now, the construction of the flow rate detector **12** in FIG. **2** will be described in detail with reference to FIG. **3** to FIG. **8****.**

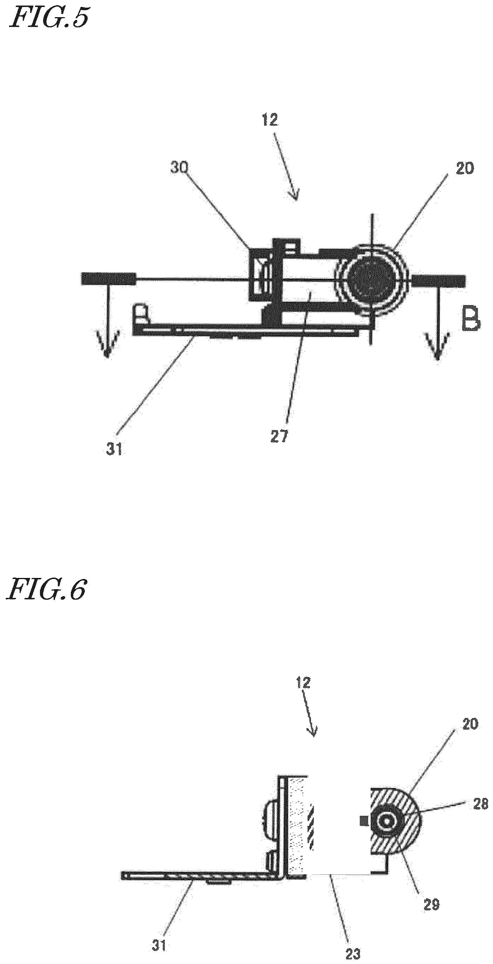

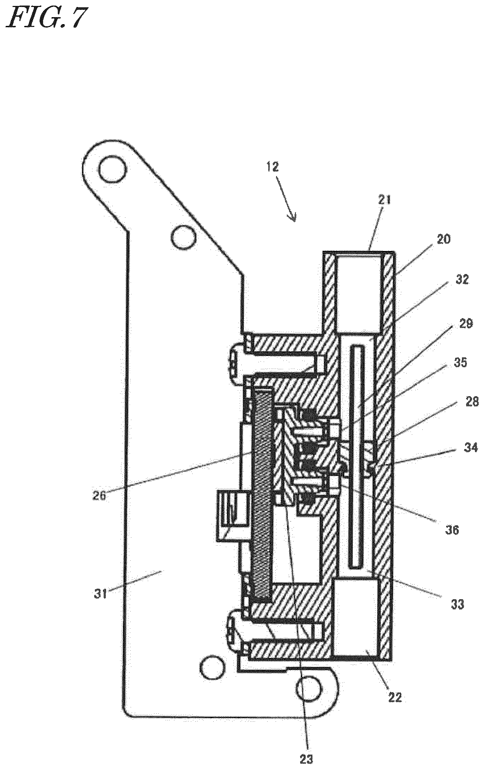

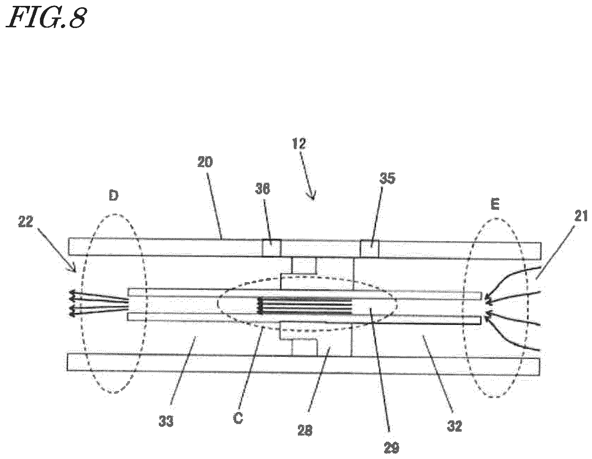

FIG. **3** is an exploded perspective view of the flow rate detector **12.** FIG. **4** is an upper plan view of the flow rate detector **12.** FIG. **5** is a side view of the flow rate detector **12.** FIG. **6** is a cross-sectional view, taken at line **A-A** and viewed in the direction of arrows in FIG. **4****.** FIG. **7** is a cross-sectional view, taken at line **B-B** and viewed in the direction of arrows in FIG. **5****.** FIG. **8** is a cross-sectional view of a main portion of the flow rate detector **12.**

As shown in FIG. **3****,** the flow rate detector **12** has a pipe body **20** made of resin, having an elongated cylindrical shape.

At a first end, the pipe body **20** has a flow inlet **21** through which exhalation from the chamber **10** (see FIG. **2**) flows in. Moreover, at a second end opposite to the first end, the pipe body **20** has a flow outlet **22** through which the exhalation having passed through the pipe body **20** flows out.

Via two O-rings **24,** a differential pressure sensor **23** is connected to the pipe body **20.** The differential pressure sensor **23** measures pressure at two places, i.e., at the flow-in side and the flow-out side of exhalation, to determine a pressure difference therebetween.

In other words, with respect to the exhalation flowing in the pipe body **20,** the exhalation measurement device **1** according to the present embodiment detects a flow rate of the exhalation, from a difference between the pressure measurements taken at the two places by the differential pressure sensor **23.**

As the differential pressure sensor **23,** a generic differential pressure sensor is employed. The differential pressure sensor **23** is provided on the substrate **26** together with other electronic parts **25.**

Moreover, two bosses **27** are provided on the pipe body **20,** near the flow inlet **21** and the flow outlet **22.**

Furthermore, as will be described in detail later, a single air duct **29** is inserted in the pipe body **20,** the air duct **29** being pressed into the center of a partition gasket **28** (which is an example of a partitioning member) having a circular columnar shape.

Then, when the bosses **27** of the flow rate detector **12** are screwed onto a metal plate **31** with two screws **30,** the flow rate detector **12** becomes assembled, as shown in FIG. **4** and FIG. **5****.** The flow rate detector **12** is disposed inside the exhalation measurement device **1.**

The inside of the pipe body **20** will be described with reference to FIG. **7****.**

As shown in FIG. **7****,** the partition gasket **28** is provided in the pipe body **20,** such that the partition gasket **28** partitions the internal space of the pipe body **20** into a flow-in space (first space) **32** on the flow inlet **21** side and a flow-out space (second space) **33** on the flow outlet **22** side.

The partition gasket **28** is a member made of rubber (as an example of an elastic body) that is pressed inside of the pipe body **20,** which is substantially cylindrical in shape. A recess which is made at an end of the partition gasket **28** that is closer to the flow outlet **22** is fitted onto an annular rib **34** which protrudes from the inner peripheral surface (inner surface) of the pipe body **20** and inwardly along the radial direction.

As a result of this, the partition gasket **28** can be restrained from moving inside the pipe body **20.**

Moreover, the single air duct **29** of metal, having an elongated cylindrical shape, is provided in the partition gasket **28,** so as to extend through the central portion of the partition gasket **28.**

The air duct **29** is a straight cylindrical member, which is pressed into the partition gasket **28** having a circular columnar shape along its axial direction.

Therefore, the internal space of the pipe body **20** is partitioned into two by the partition gasket **28,** such that the partitioned flow-in space **32** and flow-out space **33** communicate with each other via the air duct **29.** Therefore, the exhalation which has flown from the flow inlet **21** of the pipe body **20** into the flow-in space **32** passes inside the elongated air duct **29** to reach the flow-out space **33,** thus flowing out of the flow outlet **22.**

A connection aperture **35** is provided in a portion of the pipe body **20** at a side of the partition gasket **28** that is closer to the flow-in space **32.** Through the connection aperture **35,** the differential pressure sensor **23** is connected to the flow-in space **32.**

On the other hand, a connection aperture **36** is provided in a portion of the pipe body **20** at a side of the partition gasket **28** that is closer to the flow-out space **33.** Through the connection aperture **36,** the differential pressure sensor **23** is connected to the flow-out space **33.**

The differential pressure sensor **23** measures a pressure of the exhalation at each of the flow-in space **32** and the flow-out space **33,** and calculates a pressure difference therebetween.

FIG. **8** is a cross-sectional view of the pipe body **20,** showing a flow of exhalation inside the pipe body **20.**

In the exhalation measurement device **1** according to the present embodiment, as shown in FIG. **8** and as described above, the partition gasket **28** (as an example of a partitioning member) that partitions the internal space of the pipe body **20** into the flow-in space **32** and the flow-out space **33** is provided inside the pipe body **20** composing the flow rate detector **12.** Moreover, the single air duct **29** of an elongated shape that extends through the partition gasket **28** is provided.

Moreover, the air duct **29** has a first end **29a** protruding from the partition gasket **28** into the flow-in space **32.** Similarly, the air duct **29** has a second end **29b** opposite to the first end **29a** protruding from the partition gasket **28** into the flow-out space **33.**

In other words, the exhalation that has flown into the flow-in space **32** is led into the single thin and long air duct **29,** which allows the flow-in space **32** and the flow-out space **33** to communicate with each other, and passes through the air duct **29** to flow out into the flow-out space **33.**

Therefore, inside the pipe body **20,** the exhalation flowing from the flow-in space **32** to the flow-out space **33** moves via the thin and long air duct **29,** and consequently the pressure difference between the flow-in space **32** and the flow-out space **33** increases.

Specifically, the pressure at the flow-in space **32,** into which the exhalation flows, becomes higher than the pressure at the flow-out space **33,** from which the exhalation flows out via the elongated air duct **29.** As a result, the pressure difference between the flow-in space **32** and the flow-out space **33** becomes greater than conventional.

Therefore, since the increased pressure difference is measured with a sufficient accuracy by the differential pressure sensor **23,** the detection accuracy for the flow rate of the exhalation as detected by using this pressure difference can be enhanced.

As a result, the pump **13** is appropriately controlled by the control section **15** with the enhanced detection accuracy for the flow rate of the exhalation, and the exhalation is supplied to the measurement section **14** with a predetermined flow rate that is managed with a high accuracy. Thus, the measurement accuracy for exhalation can be enhanced.

In the flow-out space **33,** as indicated in Area **D** of FIG. **8****,** the exhalation that has flown out of the outlet (i.e., the end of the air duct **29** on the flow-out space **33** side) of the air duct **29** gradually expands its flow path, away from the second end **29b** at the flow-out side of the air duct **29,** this being due to a difference in inner diameter between the pipe body **20** and the air duct **29.**

Thus, when a change in the flow path of the exhalation occurs, turbulence (i.e., a flow in which the exhalation irregularly fluctuates with respect to velocity, pressure, etc.) is likely to emerge there. If turbulence emerges near the outlet of the air duct **29,** it is foreseeable that the measurement accuracy for pressure difference in the exhalation may lower under the influences of pressure fluctuations due to this turbulence.

Therefore, in the present embodiment, the exhalation is allowed to pass through the elongated air duct **29** as described above. In this respect, the exhalation which has entered the air duct **29** has its direction of flow organized by the elongated air duct **29,** and its straightness along the longitudinal direction of the air duct **29** is improved. Then, the exhalation having exited the outlet of the air duct **29** (second end **29b**) travels straight over a predetermined distance, whereby changes in the flow path of the exhalation near the outlet of the air duct **29** are suppressed.

This can restrain turbulence from emerging near the outlet of the air duct **29** (Area **D** of the flow-out space **33**).

Then, with the turbulence near the outlet (second end **29b**) of the pipe body **20** being suppressed, a pressure difference in the exhalation is measured at a position which is distant from a position where turbulence is likely to emerge (i.e., a position which is distant from the second end **29b** at the outlet side of the air duct **29**).

Specifically, in the flow-out space **33,** the air duct **29** is disposed so as to protrude from the partition gasket **28** into the flow-out space **33,** and so as to be distanced from the inner peripheral surface of the pipe body **20.** In other words, in the flow-out space **33,** a cylindrical gap **37** is created between the outer peripheral surface of the air duct **29** and the inner peripheral surface of the pipe body **20.**

The gap **37** is formed at a position which is distant from the second end **29b** at the outlet side of the air duct **29,** where turbulence is likely to emerge.

Furthermore, the differential pressure sensor **23** to measure a pressure within the flow-out space **33** communicates with the flow-out space **33** via the connection aperture **36** being provided at a position corresponding to the gap **37.**

Moreover, the connection aperture **36** is provided at a position which is closer to the partition gasket **28** than to the second end **29b** at the outlet side of the air duct **29** in the pipe body **20.** Stated otherwise, the connection aperture **36** is provided at a position of the pipe body **20** toward the center of the air duct **29.**

As a result, in the flow-out space **33,** pressure of the exhalation is measured at a position which is distant from the second end **29b** at the outlet side of the air duct **29** (where turbulence is likely to emerge), while turbulence near the outlet of the air duct **29** is suppressed. Therefore, in the flow-out space **33,** while hardly being affected by turbulence, pressure of the exhalation can be appropriately measured. Thus, the detection accuracy for the flow rate of the exhalation as detected by using the pressure difference between the flow-out space **33** and the flow-in space **32** can be enhanced.

As a result, the pump **13** is appropriately controlled by the control section **15** with an enhanced detection accuracy for the flow rate of the exhalation, and the exhalation is supplied to the measurement section **14** with a predetermined flow rate that is managed with a high accuracy, whereby the measurement accuracy for exhalation can be enhanced.

Note that, near the inlet of the air duct **29** (i.e., the first end **29a** of the air duct **29** at the flow-in space **32** side) in the flow-in space **32,** as indicated in Area **E** of FIG. **8****,** the exhalation flows into the thin air duct **29.**

In the flow-in space **32,** too, the air duct **29** is disposed so as to protrude from the partition gasket **28** into the flow-in space **32,** and so as to be distanced from the inner peripheral surface of the pipe body **20.** In other words, in the flow-in space **32,** a cylindrical gap **38** is created between the outer peripheral surface of the air duct **29** and the inner peripheral surface of the pipe body **20.**

The gap **38** is formed at a position which is distant from the first end **29a** at the inlet side of the air duct **29.**

Furthermore, the differential pressure sensor **23** to measure a pressure within the flow-out space **33** communicates with the flow-in space **32,** via the connection aperture **35** being provided at a position corresponding to the gap **38.**

Moreover, the connection aperture **35** is provided at a position which is closer to the partition gasket **28** than to the first end **29a** at the inlet side of the air duct **29** in the pipe body **20.** Stated otherwise, the connection aperture **36** is provided at a position of the pipe body **20** toward the center of the air duct **29.**

Therefore, even if turbulence emerges near the inlet of the air duct **29** (first end **29a**), pressure of the exhalation is measured at a position which is distant from the turbulence. As a result, in the flow-in space **32,** pressure of the exhalation can be appropriately measured, while hardly being affected by turbulence. Thus, the detection accuracy for the flow rate of the exhalation by using a pressure difference between the flow-in space **32** and the flow-out space **33** can be enhanced.

As a result, the pump **13** is appropriately controlled by the control section **15** with an enhanced detection accuracy for the flow rate of the exhalation, and the exhalation is supplied to the measurement section **14** with a predetermined flow rate that is managed with a high accuracy, whereby the measurement accuracy for exhalation can be improved relative to the conventional level.

As described above, in the flow-in space **32** and the flow-out space **33,** the air duct **29** is so as to be distanced from the inner peripheral surface of the pipe body **20,** and substantially cylindrical gaps **37** and **38** are formed between the outer peripheral surface of the air duct **29** and the inner peripheral surface of the pipe body **20.**

At the gaps **37** and **38,** there is less flow of exhalation (or hardly any flow of exhalation) than there is the flow of exhalation in the air duct **29,** the flows of exhalation near the inlet and outlet of the air duct **29** (Area **D** and Area **E** of FIG. **8**), and the flows of exhalation at the flow inlet **21** and the flow outlet **22** of the pipe body **20.**

Furthermore, the differential pressure sensor **23** is connected to the gap **38** of the flow-in space **32** via the connection aperture **35,** and connected to the gap **37** of the flow-out space **33** via the connection aperture **36.**

As a result, while hardly being affected by unevennesses in the flow of exhalation (e.g., turbulence), pressure difference in the exhalation between the flow inlet **21** and the flow outlet **22** is measured, whereby the detection accuracy for the flow rate of the exhalation can be further enhanced.

Moreover, according to the present embodiment, as shown in FIG. **8****,** in the flow-out space **33,** the connection aperture **36** having the differential pressure sensor **23** connected thereto is provided at a position which is closer to the partition gasket **28** than to the second end **29b** at the outlet side of the air duct **29.**

In other words, as described above, exhalation is passed through the long air duct in order to suppress turbulence which may emerge near the outlet of the air duct **29;** in order to further reduce the influences of turbulence, the connection aperture **36** is disposed at a position which is close to the partition gasket **28,** away from the second end **29b** at the outlet side of the air duct **29.**

Therefore, pressure of the exhalation at the flow-out space **33** side is measured at a position which is less susceptible to influences of turbulence, whereby a pressure difference between the flow-in space **32** and the flow-out space **33** can be measured highly accurately. As a result, the detection accuracy for the flow rate of the exhalation as detected based on a pressure difference between the flow-in space **32** and the flow-out space **33** can be enhanced.

Furthermore, in the present embodiment, as shown in FIG. **8****,** the air duct **29** is disposed so that its protruding length (i.e., distance from the partition gasket **28** to the first end **29a** or the second end **29b** of the air duct **29**) from the partition gasket **28** is greater than the length (thickness) of the partition gasket **28,** along its longitudinal direction.

In particular, in the flow-out space **33,** the air duct **29** is formed so that its protruding length from the partition gasket **28** is greater than the length (thickness)of the partition gasket **28,** along its longitudinal direction. In other words, the air duct **29** in the flow-out space **33** protrudes far out of the partition gasket **28.**

Therefore, the connection aperture **36** to which the differential pressure sensor **23** is connected can be provided at a position which is very distant from a position near the outlet of the air duct **29** where turbulence is likely to emerge (i.e., at a position which is less susceptible to influences of turbulence).

Then, the differential pressure sensor **23** is able to measure the pressure of the exhalation at the flow-out space **33** side, at a position which is less susceptible to influences of turbulence which is likely to emerge near the outlet of the air duct **29.**

Thus, since a pressure difference in the exhalation is measured by using a pressure of the exhalation that is measured at a position which is less susceptible to influences of turbulence, the detection accuracy for the flow rate of the exhalation can be further enhanced.

Note that the air duct **29** may be constructed to protrude only into the flow-out space **33.** Under this construction, too, providing the connection aperture **36** at a position which is distant from a position near the outlet of the air duct **29** where turbulence is likely to emerge allows the pressure of the exhalation to be measured without being affected by turbulence.

Furthermore, in the present embodiment, the pipe body **20** and the air duct **29** are formed so as to be substantially cylindrical, and disposed so that the center axis of the pipe body **20** and the center axis of the air duct **29** coincide (i.e., overlap). In other words, the pipe body **20** and the air duct **29** constitute a double cylinder in a so-called concentric arrangement.

Therefore, the flow of exhalation in the pipe body **20** can be created in an axisymmetric manner, as centered around the center axis of the pipe body **20** and the air duct **29.**

In particular, in the flow-out space **33,** the flow of exhalation to come out of the air duct **29** is created in an axisymmetric manner, so that the flow of exhalation is organized in the flow-out space **33,** whereby turbulence near the second end **29b** of the air duct **29** can be suppressed.

Therefore, a pressure difference in the exhalation between the flow-in space **32** and the flow-out space **33** is measured highly accurately while reducing the influences of turbulence, whereby the detection accuracy for the flow rate of the exhalation can be enhanced.

Furthermore, in the present embodiment, as shown in FIG. **8****,** the inner diameter of the air duct **29** is smaller than a half of the inner diameter of the pipe body **20.**

In other words, a cross-sectional area along a direction which is perpendicular to the axis of the air duct **29** is formed so as to be sufficiently smaller than the cross-sectional area of the pipe body.

Consequently, the exhalation flowing from the flow-in space **32** to the flow-out space **33** is passed through the thin air duct **29,** thus resulting in a large pressure difference between the exhalation in the flow-in space **32** and the exhalation in the flow-out space **33.**

Therefore, since the increased pressure difference is measured with a sufficient accuracy, the detection accuracy for the flow rate of the exhalation can be enhanced.

Furthermore, in the present embodiment, the inner peripheral surface of the air duct **29** has a smaller surface roughness than does the inner peripheral surface of the pipe body **20.**

Specifically, the air duct **29** is made of a metal (e.g., a stainless steel), whereas the pipe body **20** is made of a resin (e.g., an ABS resin).

Accordingly, the inner peripheral surface of the metal air duct **29** has a smaller surface roughness than does the inner peripheral surface of the resin pipe body **20.** Therefore, the inner peripheral surface of the air duct **29** has reduced surface irregularities than does the inner peripheral surface of the pipe body **20,** thus making it easier for the exhalation to pass through the air duct **29** having reduced surface irregularities.

Thus, the flow of exhalation can be restrained from becoming turbulent within the air duct **29,** whereby turbulence near the second end **29b** at the outlet side of the air duct **29** (Area **D** of the flow-out space **33**) can be suppressed.

Therefore, a pressure difference in the exhalation between the flow-in space **32** and the flow-out space **33** is measured while reducing the influences of turbulence, whereby the detection accuracy for the flow rate of the exhalation as detected by using this pressure difference can be further enhanced.

Furthermore, in the present embodiment, in the flow-out space **33,** the connection aperture **36** having the differential pressure sensor **23** connected thereto is provided at a position which is closer to the center of the air duct **29** (i.e., toward the partition gasket **28**) than to the second end **29b** of the air duct **29.**

In other words, in the present embodiment, exhalation is passed through the long air duct **29** in the aforementioned manner, thus enhancing straightness of the exhalation, and suppressing turbulence which may emerge near the outlet of the air duct **29;** in order to further reduce the influences of turbulence, the connection aperture **36** is provided at a position which is close to the center of the air duct **29,** away from the second end **29b** at the outlet side of the air duct **29.**

Therefore, pressure of the exhalation at the flow-out space **33** side is measured at a position which is distant from a position where turbulence is likely to emerge, whereby the detection accuracy for the flow rate of the exhalation can be further enhanced.

Furthermore, in the present embodiment, the air duct **29** is made of a single cylinder.

That is, when the air duct **29** is formed by bundling together a multitude of elongated tubes, the inner peripheral surface of each tube will have a small machining error. Before and after the air duct **29,** machining errors of a plurality of tubes will add up and increase, thus allowing the pressure of the exhalation to fluctuate. This might lower the detection accuracy for the flow rate of the exhalation.

In the exhalation measurement device **1** according to the present embodiment, the air duct **29** is made of a single cylinder, and therefore machining errors will not add up.

Thus, since the pressure difference in the exhalation is appropriately measured, the detection accuracy for the flow rate of the exhalation using this pressure difference can be further enhanced.

Note that, in the present embodiment, both of the connection aperture **35** at the inlet side of the air duct **29** and the connection aperture **36** at the outlet side of the air duct **29** are provided at positions respectively corresponding to the gaps **37** and **38** created between the outer peripheral surface of the air duct **29** and the inner peripheral surface of the pipe body **20,** as described above.

However, when the turbulence emerging near the inlet of the air duct **29** is smaller than the turbulence near the outlet of the air duct **29,** it suffices if only the connection aperture **36** at the outlet side is provided at the position corresponding to the gap **37** between the air duct **29** and the pipe body **20.**

In other words, in the case where the flow of exhalation that reaches the inlet of the air duct **29** of the flow-in space **32** is a laminar flow (i.e., a flow in which the exhalation does not irregularly fluctuate with respect to velocity, pressure, etc.), for example, the turbulence emerging near the inlet of the air duct **29** (first end **29a**) will be negligibly small relative to the turbulence emerging near the outlet (second end **29b**).

In this case, only the connection aperture **36** at the outlet (second end **29b**) side may be provided at the position corresponding to the gap **37** created between the outer peripheral surface of the air duct **29** and the inner peripheral surface of the pipe body **20.**

Thus, the exhalation measurement device **1** according to the present embodiment includes: a measurement apparatus main body **4** into which exhalation is to be blown; a measurement section **14** to take a measurement of the exhalation; a chamber **10** to temporarily retain the exhalation that has been blown into the measurement apparatus main body **4;** a pump **13** to allow the exhalation in the chamber **10** to be supplied to the measurement section **14;** a control section **15** to control operation of the pump **13;** and a flow rate detector **12** to detect a flow rate of the exhalation that is supplied by the pump **13** to the measurement section **14.**

The flow rate detector **12** includes: a pipe body **20** having a flow inlet **21** at a first end, into which the exhalation flows, and a flow outlet **22** at a second end, out of which the exhalation flows; and a differential pressure sensor **23** connected to the pipe body **20.**

The pipe body **20** includes: a partition gasket **28** (as an example of a partitioning member) which partitions the inside of the pipe body **20** into a flow-in space **32** and a flow-out space **33** for the exhalation; a connection aperture **35** being provided at the flow-in space **32** and connecting the differential pressure sensor **23** to the flow-in space **32;** a connection aperture **36** being provided at the flow-out space **33** and connecting the differential pressure sensor **23** to the flow-out space **33;** and an air duct **29** of an elongated shape extending through the partition gasket **28** and allowing the flow-in space **32** and the flow-out space **33** to communicate with each other.

Thus, in the exhalation measurement device **1** according to the present embodiment, the pipe body **20** of the flow rate detector **12** includes: the partition gasket **28,** which partitions the inside into the flow-in space **32** and the flow-out space **33** for the exhalation; and the air duct **29** of an elongated shape, extending through the partition gasket **28** and allowing the flow-in space **32** and the flow-out space **33** to communicate with each other.

Therefore, inside the pipe body **20,** the exhalation passes through the thin and long air duct **29** in moving over to the flow-out space **33** side, thus inducing a large pressure difference in the exhalation between the flow-in space **32** and the flow-out space **33.**

Accordingly, a pressure difference which has been made greater than conventional is measured with a sufficient accuracy, whereby the detection accuracy for the flow rate of the exhalation as detected by using this pressure difference can be enhanced.

As a result, the pump **13** is appropriately controlled so as to attain a flow rate of the exhalation which is highly accurately managed on the basis of the accurately detected pressure difference, whereby the measurement accuracy of the concentration of nitrogen monoxide that is contained in the exhalation can be enhanced.

### INDUSTRIAL APPLICABILITY

The present invention is expected to find use in an exhalation measurement device to be used when e.g. detecting asthma, or checking pulmonary function.

### REFERENCE SIGNS LIST

- **1**: exhalation measurement device
- **2**: handle section
- **3**: tube
- **4**: measurement apparatus main body
- **5**: handle section main body
- **6**: mouthpiece
- **7**: exhalation opening
- **8**: pressure sensor
- **9**: flow rate adjuster
- **10**: chamber
- **11**: input gas switching device
- **12**: flow rate detector
- **13**: pump
- **14**: measurement section
- **15**: control section
- **16**: display section
- **17**: power switch
- **18**: memory
- **19**: zero gas generator
- **20**: pipe body
- **21**: flow inlet
- **22**: flow outlet
- **23**: differential pressure sensor
- **24**: O-ring
- **25**: electronic parts
- **26**: substrate
- **27**: boss
- **28**: partition gasket (partitioning member)
- **29**: air duct
- **29a**: first end
- **29b**: second end
- **30**: screw
- **31**: metal plate
- **32**: flow-in space (first space)
- **33**: flow-out space (second space)
- **34**: rib
- **35**: connection aperture
- **36**: connection aperture
- **37**: gap
- **38**: gap

## Claims

1. An exhalation measurement device comprising:
a measurement apparatus main body into which exhalation is to be blown;
a measurement section to take a measurement of the exhalation;
a chamber to temporarily retain the exhalation that has been blown into the measurement apparatus main body;
a pump to allow the exhalation in the chamber to be supplied to the measurement section;
a control section to control operation of the pump; and
a flow rate detector to detect a flow rate of the exhalation that is supplied by the pump to the measurement section, wherein,
the flow rate detector includes
a pipe body having a flow inlet at a first end, into which the exhalation flows, and a flow outlet at a second end opposite to the first end, out of which the exhalation flows, and
a differential pressure sensor connected to the pipe body; and
the pipe body includes
a partitioning member which partitions an inside of the pipe body into a first space at a flow-in side of the exhalation and a second space at a flow-out side of the exhalation,
a first connection aperture being provided at the first space side and connecting the differential pressure sensor to the first space,
a second connection aperture being provided at the second space side and connecting the differential pressure sensor to the second space, and
an air duct of an elongated shape extending through the partitioning member and allowing the first space and the second space to communicate with each other.

2. The exhalation measurement device of claim 1, wherein,
in the second space,
the air duct is disposed so as to be distanced from an inner surface of the pipe body and protruding from the partitioning member, and
the second connection aperture is disposed at a position corresponding to a gap created between an outer surface of the air duct and the inner surface of the pipe body.

3. The exhalation measurement device of claim 2, wherein,
in the first space,
the air duct is disposed so as to be distanced from the inner surface of the pipe body and protruding from the partitioning member, and
the first connection aperture is disposed at a position corresponding to a gap created between the outer surface of the air duct and the inner surface of the pipe body.

4. The exhalation measurement device of claim 2 or 3, wherein,
in the second space,
the second connection aperture is provided at a position which is closer to the partitioning member than to an end of the air duct along a longitudinal direction thereof.

5. The exhalation measurement device of any of claims 1 to 4, wherein,
in the second space,
the air duct is formed so that, along a longitudinal direction thereof, a protruding length from the partitioning member is greater than a length of the partitioning member.

6. The exhalation measurement device of any of claims 1 to 5, wherein,
the pipe body is formed so as to be substantially cylindrical;
the air duct is formed so as to be substantially cylindrical; and
a center axis of the pipe body and a center axis of the air duct are disposed so as to overlap each other.

7. The exhalation measurement device of claim 6, wherein an inner diameter of the air duct is smaller than a half of an inner diameter of the pipe body.

8. The exhalation measurement device of any of claims 1 to 7, wherein an inner surface of the air duct has a smaller surface roughness than does an inner surface of the pipe body.

9. The exhalation measurement device of any of claims 1 to 8, wherein,
in the second space,
the second connection aperture is provided at a position which is closer to the center of the air duct than to an end of the air duct along a longitudinal direction thereof.

10. The exhalation measurement device of any of claims 1 to 9, wherein the air duct is made of a single cylinder.
